(19) Europäisches Patentamt
European Patent Office
Office européen des brevets

(11) **EP 1 263 444 B1**

(12) # FASCICULE DE BREVET EUROPEEN

(45) Date de publication et mention de la délivrance du brevet:
**27.07.2005 Bulletin 2005/30**

(21) Numéro de dépôt: **01907785.8**

(22) Date de dépôt: **14.02.2001**

(51) Int Cl.[7]: **A61K 31/675**, A61P 19/10

(86) Numéro de dépôt international:
**PCT/FR2001/000421**

(87) Numéro de publication internationale:
**WO 2001/060372 (23.08.2001 Gazette 2001/34)**

(54) **APPLICATION DE DERIVES DE XANTHINE POUR LA PREPARATION D'UN MEDICAMENT DESTINE A LA PREVENTION OU AU TRAITEMENT DE L'OSTEOPOROSE**

VERWENDUNG VON XANTHINEDERIVATEN ZUR HERSTELLUNG EINES ARZNEIMITTELS ZUR BEHANDLUNG ODER VERHÜTUNG VON OSTEOPOROSE

USE OF XANTHINE DERIVATIVES FOR PREPARING A MEDICINE FOR PREVENTING AND TREATING OSTEOPOROSIS

(84) Etats contractants désignés:
**AT BE CH CY DE DK ES FI FR GB GR IE IT LI LU MC NL PT SE TR**

(30) Priorité: **15.02.2000 FR 0001846**

(43) Date de publication de la demande:
**11.12.2002 Bulletin 2002/50**

(73) Titulaire: **Aventis Pharma S.A.**
**92160 Antony (FR)**

(72) Inventeurs:
- **BILLEN, Günter, Johannes**
  **65527 Niedernhausen (DE)**
- **BOUALI, Yamina**
  **F-94800 Villejuif (FR)**
- **SATOH, Yusuke**
  **Kawagoe-shi, Saitama 350-1173 (JP)**
- **VEVERT, Jean-Paul**
  **F-93500 Pantin (FR)**

(74) Mandataire: **Rousseau, Pierrick Edouard**
**Aventis Pharma S.A.**
**Direction des Brevets,**
**Tri LEO/144**
**20, avenue Raymond Aron**
**92165 Antony Cedex (FR)**

(56) Documents cités:
**EP-A- 0 812 844** **US-A- 5 629 315**
**US-A- 5 728 686**

- **PATENT ABSTRACTS OF JAPAN vol. 10 (C, 31 octobre 1997 (1997-10-31) & JP 09 169665 A (MIYAMOTO ET AL), 30 juin 1997 (1997-06-30)**
- **PATENT ABSTRACTS OF JAPAN vol. 09 (C, 30 juillet 1999 (1999-07-30) & JP 11 092379 A (HOECHST MARION ROUSSEL KK), 6 avril 1999 (1999-04-06)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention a pour objet l'application de dérivés de xanthine pour la préparation d'un médicament destiné à la prévention ou au traitement de l'ostéoporose.

L'ostéoporose est une pathologie diffuse du squelette caractérisée par une diminution de la masse osseuse (ostéopénie) à laquelle vient se rajouter une désorganisation de la microarchitecture de l'os conduisant à une diminution de la résistance mécanique de l'os et à une augmentation du risque fracturaire (ostéoporose).

L'ostéoporose est une maladie multifactorielle. C'est la ménopause, naturelle ou chirurgicale qui chez la femme constitue le facteur de risque primordial.

La perte osseuse post-ménopausique et l'ostéoporose, maladie qui en en est la conséquence, résultent d'un déséquilibre du remodelage osseux au profit de la résorption osseuse par les ostéoclastes.

Cette ostéopénie est accélérée après la ménopause, du fait de la carence estrogénique, ce qui explique la large prédominance féminine de cette maladie.

Il en résulte une raréfaction progressive du tissu osseux, aboutissant à une fragilisation osseuse dont la conséquence clinique est la survenue de fractures spontanées.

L'estrogénothérapie est le traitement médicamenteux préventif de première intention des fractures ostéoporotiques, malgré les incertitudes concernant notamment les risques cancérologiques éventuels (sein et endomètre) ce qui limite son application.

Il convient donc de trouver des produits capables de prévenir la perte osseuse postménopausique en cas de contre indication ou de « non désir » du traitement estrogénique.

Jusqu'à présent, seuls des inhibiteurs de la résorption osseuse (SERMs (Selective Estrogen Receptors Modulators)), les biphosphonates, ou la calcitonine sont disponibles pour cette thérapeutique. Un des objectifs de la présente invention est par conséquent de mettre au point des substances capables de stimuler la formation osseuse, d'augmenter réellement la masse osseuse et de restaurer la résistance mécanique.

L'invention a donc pour objet l'application d'un dérivé de xanthine de formule (I) :

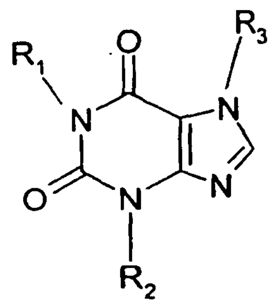

(I)

ainsi que ses sels physiologiquement acceptables pour la préparation d'un médicament destiné à la prévention ou au traitement de l'ostéoporose, dans laquelle $R_1$ et $R_3$ sont identiques ou différents, et au moins l'un des radicaux $R_1$ et $R_3$ représente :

a) un radical de formule F1

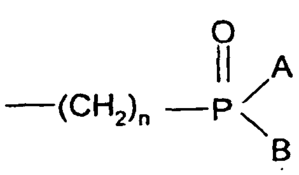

(F1)

dans laquelle A et B identiques ou différents, indépendamment l'un de l'autre représentent $C_{1-4}$ alkyle, $C_{1-6}$ alkoxy, hydroxyle ou benzyloxy et n représente un entier de 1 à 4,

b) si un seul des radicaux $R_1$ ou $R_3$ présente la signification donnée en a), l'autre radical $R_1$ ou $R_3$ est

1) un atome d'hydrogène
2) un radical $C_{1-6}$ alkyle

3) un radical $C_{2-6}$ alkyle interrompu par un atome d'oxygène
4) un radical $C_{3-8}$ cycloalkyle-
5) un radical $C_{3-8}$ cycloalkyl-$C_{1-4}$ alkyle-
6) un radical $C_{2-6}$ alkényle ou
7) un radical benzyle

$R_2$ représente

1) un atome d'hydrogène,
2) un $C_{1-6}$ alkyle
3) un $C_{2-6}$ alkényle
4) un phényle,
5) un benzyle,
6) un $C_{3-8}$ cycloalkyle, ou
7) un $C_{3-8}$ cycloalkyl-$C_{1-4}$ alkyle-.

Les radicaux alkyles peuvent être linéaires ou ramifiés. La définition englobe évidemment toutes les formes stéréo-isomériques, seules ou en mélanges, des composés de formule (I)

Les composés de formule (I) sont décrits et préparés dans le brevet US 5,728,686 (17 mars 1998). Ils sont connus pour le traitement de tous désordres causés par l'augmentation de la production de TNF (tumor necrosis factor) ou un dérèglement de cette production. Les applications thérapeutiques sont nombreuses telle que le traitement ou la prévention de l'atrophie musculaire, la dystrophie musculaire, la pneumonie chronique.

La demanderesse a mis en évidence une nouvelle application thérapeutique des composés de formule (I).

Les composés de formule (I) peuvent ainsi être utilisés à titre de médicaments dans le traitement ou la prévention des maladie de l'os.

L'invention a plus particulièrement pour objet l'application telle que définie précédemment dans laquelle :

- $R_1$ représente un radical FI tel que défini plus haut avec

  . n égal à 1,
  . A et B, identiques ou différents, représentent un radical $C_{1-4}$ alkyle,

- $R_2$ est tel que défini plus haut et
- $R_3$ est un atome d'hydrogène.

L'invention a tout particulièrement pour objet l'application telle que définie précédemment d'un composé de formule (I) dont le nom suit :

[1-(3-phénylxanthin-1-yl)méthyl]diméthylphosphine oxide.

Les sels physiologiquement acceptables des composés de formule (I) sont en particulier des sels pharmaceutiquement utilisables ou non toxiques ou physiologiquement utilisables.

Lorsque les composés de formule (I) contiennent un groupe basique, ils peuvent former un sel d'addition avec les acides par exemple avec les acides inorganiques tels que l'acide chlorhydrique, sulfurique, phosphorique ou avec les acides organiques carboxyliques tels que l'acide acétique, trifluoroacétique, citrique, benzoïque, maléique, fumarique, tartrique, méthanesulfonique ou para toluène sulfonique.

Ils peuvent être administrés tels quels ou en mélange avec un ou plusieurs autres composés de formule (I) ou encore sous la forme d'une préparation pharmaceutique (composition pharmaceutique) qui permet une administration entérale ou parentérale et qui renferme à titre de composé actif une dose efficace d'au moins un composé de formule (I) et/ou ses sels physiologiquement acceptables et/ou ses prodrugs ainsi que des supports et/ou additifs courants et pharmaceutiquement inertes.

Les compositions pharmaceutiques qui permettent une administration entérale ou parentérale renferment à titre de composé actif une dose efficace d'au moins un composé de formule (I) et/ou ses sels physiologiquement acceptables et/ou ses prodrugs ainsi qu'un ou plusieurs supports ou véhicules pharmaceutiquement inertes, et le cas échéant un ou plusieurs additifs usuels.

Les médicaments peuvent être administrés oralement, par exemple sous forme de pilule, de comprimés, de comprimés enrobés, de pelliculés, de granules, de gélules et capsules molles, de solutions, de sirops, d'émulsions, de suspensions ou de mélanges d'aérosols.

L'administration peut cependant être effectuée par voie rectale, par exemple sous forme de suppositoire ou par voie parentérale, par exemple sous forme de solutions injectables ou d'infusions, de microcapsules ou d'implants, ou par voie percutanée, par exemple sous la forme de pommade, de solutions, de pigments ou de colorants, ou par d'autre voie telle que sous la forme d'aérosol ou de spray nasal.

Les compositions pharmaceutiques sont préparées selon des méthodes connues en soi, des supports organiques ou inorganiques, pharmaceutiquement inertes étant additionnés aux composés de formule (I) et/ou leurs sels physiologiquement acceptables et/ou leurs prodrugs.

Pour la production de pilules, de comprimés, de comprimés enrobés et de capsules en gélatine dure, il est possible d'utiliser par exemple, du lactose, de l'amidon de maïs ou ses dérivés, du talc, de l'acide stéarique ou ses sels. Les supports convenables pour des capsules en gélatine molle ou pour les suppositoires sont par exemple les graisses, les cires, les polyols semi-solides ou liquides, les huiles naturelles ou modifiées. Les véhicules appropriés pour la préparation de solutions, par exemple les solutions injectables, les émulsions ou les sirops sont par exemple l'eau, les alcools, le glycérol, les polyols, le sucrose, les sucres invertis, le glucose, les huiles végétales. Les supports convenables pour les microcapsules ou les implants sont par exemple les copolymères d'acide glyoxylique et d'acide lactique. Les préparations pharmaceutiques contiennent normalement de 0,5 % à 90 % en poids de composés de formule (I) et/ou leurs sels physiologiquement acceptables.

En plus des principes actifs et des supports, les préparations pharmaceutiques peuvent contenir des additifs tels que par exemple des diluants, des désintégrants, des liants, des lubrifiants, des agents mouillant, des stabilisants, des émulsifiants, des préservateurs, des agents sucrants, des colorants des agents de flaveurs ou des aromatisants, des épaississants, des agents tampons, et aussi des solvants ou des solubilisants ou des agents pour obtenir un effet retard et également des sels pour modifier la pression osmotique, des agents d'enrobage ou des antioxydants.

Elles peuvent également contenir deux ou plusieurs composés de formule (I) et/ou leurs sels physiologiquement acceptables et/ou leurs prodrugs. En outre, en plus d'au moins un ou plusieurs composés de formule (I) et/ou leurs sels physiologiquement acceptables et/ou leurs prodrugs, ils peuvent contenir au moins un ou plusieurs autres principes actifs utilisables à titre thérapeutique ou prophylactique.

Les préparations pharmaceutiques (compositions pharmaceutiques) renferment normalement de 0,2 à 500 mg, et de préférence de 1 à 200 mg de composé de formule (I) et/ou leurs sels physiologiquement acceptables et/ou leurs prodrugs.

L'action des composés de formule (I) peut être démontrée par exemple dans un test dans lequel on évalue l'impact osseux du dérivé de xanthine sur un modèle d'ostéopénie induite sur une rate ovariectomisée.

Les maladies de l'os dont le traitement ou la prévention nécessitent l'emploi des composés de formule (I), sont notamment l'ostéoporose post ménopausique, l'ostéoporose chez l'homme, l'hypercalcémie, l'ostéopénie, par exemple causée par les métastases osseuses, les désordres dentaires par exemple les parodontites, l'hyperparathyroïdisme, les érosions périarticulaires dans l'arthrite rhumatoïde, et la maladie de Paget. En outre les composés de formule (I) peuvent être utilisés pour soulager, empêcher ou traiter les désordres de l'os qui sont causés par les traitements, par les glucocorticoïdes, les thérapies liées à la prise de stéroïdes ou de corticostéroïdes (ostéoporose secondaire telles que l'ostéoporose cortisonique), les ostéoporoses liées à l'immobilisation ou par les déficiences d'hormones sexuelles mâles ou femelles.

Les composés de formule (I) peuvent également être utiles pour la reconstruction osseuse ou la consolidation des fractures osseuses.

Tous ces désordres sont caractérisés par une perte osseuse, qui est basée par un défaut d'équilibre entre la formation osseuse et la destruction osseuse et qui peut être influencé favorablement par l'inhibition de la résorption osseuse par les ostéoclastes.

Lorsqu'on utilise les composés de formule (I), les doses peuvent varier à l'intérieur de limites larges et doivent être fixées en fonction de la personne à traiter. Ceci dépend par exemple du composé employé ou de la nature et de la sévérité de la maladie à traiter et si on se trouve dans des conditions graves ou chroniques ou si on met en oeuvre un traitement prophylactique.

Dans le cas d'une administration par voie orale, la dose quotidienne varie en général de 0,01 à 100 mg/kg et de préférence de 0,1 à 50 mg/kg, en particulier de 0,1 à 5 mg/kg.

Dans le cas d'une administration par voie intraveineuse, la dose quotidienne varie approximativement de 0,01 à 100 mg/kg et de préférence de 0,05 à 10 mg/kg.

La dose quotidienne peut être divisée, en particulier dans le cas de l'administration de grande quantité de principe actif, en plusieurs, par exemple 2, 3 ou 4 parts. Le cas échéant, en fonction du comportement individuel, il peut être nécessaire d'administrer les différentes doses de manière croissante ou décroissante.

## Tests pharmacologiques

L'impact osseux des dérivés de xanthine selon l'invention a été évalué sur un modèle d'ostéopénie, la rate

ovariectomisée à l'âge de 3 mois, afin de déterminer son effet sur la masse osseuse, et sur l'activité de formation et de résorption osseuse sur ce modèle. Les animaux sont traités en préventif.

| **Animaux :** | |
|---|---|
| Espèce | rat |
| Souche | Sprague-Dawley |
| Sexe | femelle |
| Poids | 250 g à 300 g |
| Nbre d'animaux/groupe | 8 |

**Produits :**

Sur ce modèle d'ostéopénie l'effet d'un dérivé xanthine est comparé à celui de la PTH

- Produit à tester : Le [1-(3-phénylxanthin-1-yl)méthyl] diméthylphosphine oxide

  - véhicule(s) : méthylcellulose 0,5 % ou sérum physiologique
  - dose(s) : 10 mg/kg pour le produit.
    la PTH est administrée à la dose de 10 et 25 µg/kg/j par voie sous cutanée
  - nombre d'administrations : une fois/jour ; 5 jours/semaine pendant 4 semaines
  - voie d'administration : voie orale pour le produit. La PTH est administrée par voie sous cutanée
  - volume : 1 ml/kg p o et s c
  - délai entre l'injection et le sacrifice : 24 heures
  - nombre d'administrations : 20.

**Chirurgie :**

Des rats femelles âgées de 3 mois et pesant environ 250-300 g sont ovariectomisées sous anesthésie à l'Imalgène 1000, à la dose de 100 mg/kg par voie intrapéritonéale (i.p) et sous un volume de 1 ml/kg. Ils reçoivent également du Nembutal (3 mg/kg i.p. sous un volume de 0,3 ml/kg). Les animaux sont répartis en groupes de 8 rats.
Après incision latérale, les plans cutanés et musculaires sont sectionnés. L'exérèse de chaque ovaire se fait après ligature de l'oviducte.
Les rats "SHAM" sont anesthésiés dans les mêmes conditions. Après incision des plans cutanés et musculaires, chaque ovaire est exposé puis replacé in situ.

**Traitements :**

Les effets des produits sont déterminés après un traitement préventif. Les traitements débutent 24 heures après ovariectomie. Les animaux sont traités pendant 4 semaines, et se répartissent de la façon suivante :

- un groupe témoin Sham recevant le ou les véhicules
- un groupe témoin OVX recevant le ou les véhicules
- OVX + dérivé xanthine
- OVX + PTH.

**Prélèvements sanguins :**

Au terme des 4 semaines de traitement, les animaux sont décapités par guillotine. Les sérums recueillis après centrifugation sont conservés à -20°C.
Un bilan lipidique sera établi à partir des dosages sériques du cholestérol total, des triglycérides et des phospholipides sur une aliquote de sérum de 500 µl.
La baisse du taux de cholestérol sérique est exprimée en % par rapport au taux présenté par les animaux ovariectomisés ne recevant que le solvant.

**Prélèvements d'organes :**

Après sacrifice des animaux, les organes suivants sont prélevés :

- tractus génital
  Les utérus sont prélevés. Ces derniers sont pesés. L'augmentation du poids est exprimée, en % du poids de l'utérus des animaux ovariectomisés ne recevant que le solvant.
- au niveau osseux :
  La masse osseuse (BMD ou Bone Mineral Density = densité minérale osseuse) est mesurée par absorptiométrie biphotonique à rayons X à double énergie (DEXA). Les mesures sont réalisées sur les os excisés et débarrassés de tous les tissus mous. La BMD (Bone Minéral Density) est mesurée sur l'os entier ainsi que sur la partie métaphysaire au niveau de l'extrémité proximale pour le tibia gauche. Cette zone est définie comme étant la région la plus riche en os trabéculaire ; et par conséquent, est la plus sensible aux variations de volume osseux et de densité minérale osseuse.

Les résultats sont exprimés en % selon la formule :

$$\frac{\text{BMD traitement testé - BMD OVX}}{\text{BMD SHAM -BMD OVX}} \times 100$$

| | Dose | OS TIBIA | UTERUS | Cholestérol |
|---|---|---|---|---|
| | mg/kg | Densité % | Poids % | % |
| Dérivé xanthine | 10 mg/kg po | 40 % | 10 % | Pas d'effet |
| PTH | 10 µg/kg | 69 % | Pas d'effet | Pas d'effet |
| PTH | 25 µg/kg sc | 100 % | 9 % | Pas d'effet |
| OVX | Véhicule(s) | 0 % | | |

Conclusion :

Utilisé à la dose de 10 mg/kg sur le modèle de la rate ovariectomisée ce produit offre une protection osseuse partielle mais significative (40 %), en comparaison de celle induite par la PTH (69 % et 100 % de protection osseuse pour respectivement les doses de 10 µg et 25 µg/kg par voie s.c.).

Ce produit ne présente en outre aucune activité utérotrophique, et pas d'effet sur le taux sérique de cholestérol. Ce produit peut être utilisé comme médicament dans la prévention ou le traitement des ostéoporoses.

## Revendications

1. Application d'un dérivé de xanthine de formule (I)

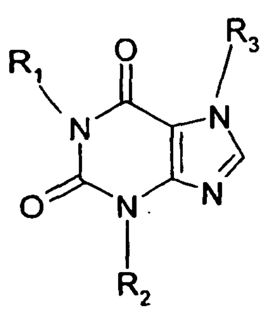

(I)

ainsi que ses sels physiologiquement acceptables pour la préparation d'un médicament destiné à la prévention ou au traitement de l'ostéoporose, dans laquelle $R_1$ et $R_3$ sont identiques ou différents, et au moins l'un des radicaux

$R_1$ et $R_3$ représente

a) un radical de formule F1

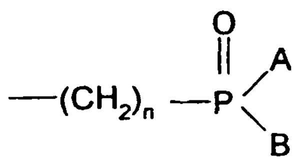

(F1)

dans laquelle A et B identiques ou différents, indépendamment l'un de l'autre représentent $C_{1-4}$ alkyle, $C_{1-6}$ alkoxy, hydroxyle ou benzyloxy et n représente un entier de 1 à 4,

b) si un seul des radicaux $R_1$ ou $R_3$ présente la signification donnée en a), l'autre radical $R_1$ ou $R_3$ est

1) un atome d'hydrogène
2) un radical $C_{1-6}$ alkyle
3) un radical $C_{2-6}$ alkyle interrompu par un atome d'oxygène
4) un radical $C_{3-8}$ cycloalkyle-
5) un radical $C_{3-8}$ cycloalkyl-$C_{1-4}$ alkyle-
6) un radical $C_{2-6}$ alkényle ou
7) un radical benzyle

$R_2$ représente :

1) un atome d'hydrogène,
2) un $C_{1-6}$ alkyle
3) un $C_{2-6}$ alkényle
4) un phényle,
5) un benzyle,
6) un $C_{3-8}$ cycloalkyle, ou
7) un $C_{3-8}$ cycloalkyl-$C_{1-4}$ alkyle-.

**2.** Application selon la revendication 1 des composés de formule (I) telle que définie à la revendication 1 dans laquelle :

- $R_1$ représente un radical F1 tel que défini à la revendication 1 avec

  . n égal à 1
  . A et B, identiques ou différents, représentent un radical $C_{1-4}$ alkyle et

- $R_2$ est tel que défini à la revendication 1,
- $R_3$ est un atome d'hydrogène.

**3.** Application selon la revendication 1 d'un composé de formule (I) telle que définie à la revendication 1 dont le nom suit :

[1-(3-phénylxanthin-1-yl)méthyl]diméthylphosphine oxide.

**Patentansprüche**

**1.** Verwendung eines Xanthinderivates der Formel (I)

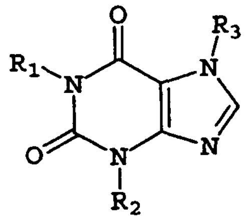

(I)

sowie seiner physiologisch akzeptablen Salze für die Herstellung eines Arzneimittels, das zur Vorbeugung oder Behandlung von Osteoporose vorgesehen ist,

worin $R_1$ und $R_3$, gleich oder verschieden, und mindestens einer der Reste $R_1$ und $R_3$ darstellen

a) einen Rest der Formel (F1)

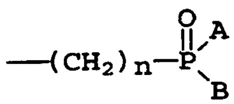

(F1)

worin A und B, gleich oder verschieden, unabhängig voneinander $C_{1-4}$-Alkyl, $C_{1-6}$-Alkoxy, Hydroxyl oder Benzyloxy darstellen und n eine ganze Zahl von 1 bis 4 bedeutet,
b) wenn einer der Reste $R_1$ oder $R_3$ die oben unter a) angegebene Bedeutung besitzt, der andere Rest $R_1$ oder $R_3$ darstellt

1) ein Wasserstoffatom
2) einen Rest $C_{1-6}$-Alkyl
3) einen Rest $C_{2-6}$-Alkyl, unterbrochen durch ein Sauerstoffatom
4) einen Rest $C_{3-8}$-Cycloalkyl-
5) einen Rest $C_{3-8}$-Cycloalkyl-$C_{1-4}$-Alkyl-
6) einen Rest $C_{2-6}$-Alkenyl oder
7) einen Rest Benzyl

$R_2$ darstellt:

1) ein Wasserstoffatom
2) einen Rest $C_{1-6}$-Alkyl
3) einen Rest $C_{2-6}$-Alkenyl
4) einen Rest Phenyl
5) einen Rest Benzyl
6) einen Rest $C_{3-8}$-Cycloalkyl oder
7) einen Rest $C_{3-8}$-Cycloalkyl-$C_{1-4}$-Alkyl-.

**2.** Verwendung nach Anspruch 1 der Verbindungen der Formel (I) wie in Anspruch 1 definiert, worin

- $R_1$ einen Rest F1 darstellt wie in Anspruch 1 definiert, mit

  . n gleich 1
  . A und B, gleich oder verschieden, die einen Rest $C_{1-4}$-Alkyl darstellen, und

- $R_2$ wie in Anspruch 1 definiert ist,
- $R_3$ ein Wasserstoffatom ist.

**3.** Verwendung nach Anspruch 1 einer Verbiridung der Formel (I) wie in Anspruch 1 definiert, deren Namen folgt:

[1-(3-Phenylxanthin-1-yl)-methyl]-dimethylphosphin-oxid.

## Claims

**1.** Application of a xanthine derivative of formula (I)

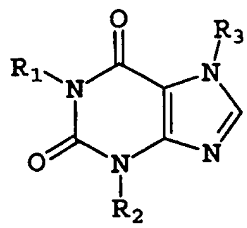

(I)

and its physiologically acceptable salts in the preparation of a medicament intended for the prevention or the treatment of osteoporosis, in which $R_1$ and $R_3$ are identical or different and at least one of the $R_1$ and $R_3$ radicals represents

a) a radical of formula F1

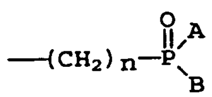

(F1)

in which A and B, which are identical or different, represent, independently of one another, $(C_{1-4})$-alkyl, $(C_{1-6})$-alkoxy, hydroxyl or benzyloxy and n represents an integer from 1 to 4,
b) if just one of the $R_1$ or $R_3$ radicals has the meaning given in a), the other $R_1$ or $R_3$ radical is

1) a hydrogen atom,
2) a $(C_{1-6})$-alkyl radical,
3) a $(C_{2-6})$-alkyl radical interrupted by an oxygen atom,
4) a $(C_{3-8})$-cycloalkyl radical,
5) a $(C_{3-8})$-cycloalkyl-$(C_{1-4})$-alkyl radical,
6) a $(C_{2-6})$-alkenyl radical or
7) a benzyl radical,

$R_2$ represents:

1) a hydrogen atom,
2) a $(C_{1-6})$-alkyl,
3) a $(C_{2-6})$-alkenyl,
4) a phenyl,
5) a benzyl,
6) a $(C_{3-8})$-cycloalkyl or
7) a $(C_{3-8})$-cycloalkyl-$(C_{1-4})$alkyl.

**2.** Application according to Claim 1 of the compounds of formula (I) as defined in Claim 1, in which:

- $R_1$ represents an F1 radical as defined in Claim 1 with
  - n equal to 1,
  - A and B, which are identical or different, representing a $(C_{1-4})$-alkyl radical, and
- $R_2$ is as defined in Claim 1,
- $R_3$ is a hydrogen atom.

**3.** Application according to Claim 1 of a compound of formula (I) as defined in Claim 1, the name of which follows:

[1-(3-phenylxanthin-1-yl)methyl)dimethylphosphine oxide.